# EUROPEAN PATENT APPLICATION

(11) **EP 4 847 488 A1**
(43) Date of publication of application: **02.12.2026**
(21) Application number: 24920040.3
(22) Date of filing: 29.11.2024
(51) Int. Cl.: C07C 231/08, C07C 233/01, C07C 233/05, C07C 233/06, C07C 233/07, C07C 1/04, B01J 23/46

(54) **METHOD FOR PRODUCING ALIPHATIC AMIDES**

(30) Priority: 25.01.2024 ES 202430057
(71) Applicant: CONSEJO SUPERIOR DE INVESTIGACIONES CIENTIFICAS (CSIC), 28006 Madrid (ES); Universitat Politécnica de Valencia, 46022 Valencia (ES)
(72) Inventor: PRIETO GONZÁLEZ, Gonzalo, 46022 Valencia Valencia (ES); CETERONI, Ilaria, 46022 Valencia Valencia (ES); ALDEGHERI, Fausto, 46022 Valencia Valencia (ES)
(74) Representative: Isern Patentes y Marcas S.L.
(86) International application number: PCT/ES2024/070755
(87) International publication number: WO 2025/158090

(57) **Abstract**

The present invention relates to a method for producing N-substituted alkylamides, including fatty N-substituted alkylamides, by means of contacting synthesis gas and an amide compound with a ruthenium catalyst.

## Description

### FIELD OF THE INVENTION

The present invention relates to a method for producing N-substituted alkylamides, including fatty N-substituted alkylamides, by means of contacting synthesis gas and an amide compound with a catalyst.

### BACKGROUND

N-substituted aliphatic amides (or simply N-alkylamides) are chemical compounds with a general molecular formula: 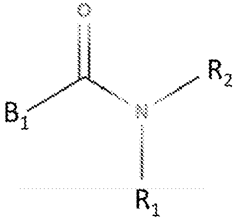 where B₁ can be a hydrogen atom or a hydrocarbyl, and at least one of the substituent groups R₁ and R₂ directly bonded to the central nitrogen atom is an alkyl group. As a particular class, fatty N-alkylamides are alkylamides in which at least one of the substituents R₁ and R₂ is an alkyl chain with *n* carbon atoms (C*ₙ*) with *n* between approx. 7 and approx. 30 carbon atoms. N-alkylamides and derivatives thereof are valuable as intermediates in the pharmaceutical and polymer industries, phase change materials, precursors of surfactant substances in different industries, including the manufacture of paints, cosmetics or the textile industry, anti-static agents, anti-caking agents, as well as precursors of alkylisonitriles and amines, among other applications.

N-alkylamides are conventionally produced by the condensation of carboxylic acids and alkylamines. Patent document US 5,681,971 describes a method for synthesising N-alkylamides from glycerides and amines in the absence of a catalyst. The document Eric Valeur and Mark Bradley, "Amide bond formation: beyond the myth of coupling reagents" Chemical Society Reviews, 2009, volume 38, pages 606-631 provides a review of the catalysts and conditions for the synthesis of N-alkylamides from carboxylic acid and amine compounds.

N-alkylamines can also be synthesised by means of catalytic amidation of aliphatic olefins with nitriles. Document US 3,530,153 presents a method for preparing N-monosubstituted secondary alkylamides. This process consists of making an aliphatic olefin react with a nitrile and water in the presence of hydrogen fluoride. The document by K. Yasude & Y. Obora, "NbCl5-mediated amidation of olefins with nitriles to secondary amides" Journal of Organometallic Chemistry, volume 775, 2015, pages 33-38 reports a method for producing N-alkylamides by amidation of olefins with nitriles.

Alternatively, N-alkylamides can be produced by means of the catalytic coupling of aliphatic alcohol or alkyl halide type reactants with amines, as described, for example, in C. Gunanathan and D. Milstein," "Applications of Acceptorless Dehydrogenation and Related Transformations in Chemical Synthesis", Science, 2013, 341, 249, or in C. L. Allen and J. M. J. Williams, "Metal-catalysed approaches to amide bond formation", Chemical Society Reviews, 2011, volume 40, 3405.

The catalytic oxidative amidation of aldehydes with alkylamide salts is another known method for producing N-alkylamides, as described in Gaspa et al. "Recent developments in oxidative esterification and amidation of aldehydes", Tetrahedron Letters, volume 57, number 31, 2016, pages 3433-3440.

A common element of the above methods for preparing N-alkylamides is that the alkyl substituents (R₁ and/or R₂) in the final N-alkylamide product must be provided in the form of a preformed *Cₙ* synthon as part of a reactant compound. Said reactant compound which, as described above, can be an aliphatic olefin, alcohol, amine, alkyl halide, etc, must be produced in a separate process or must be isolated from natural sources. It is desirable to develop methods for producing alkylamides in which the alkyl substituents can be produced from monocarbon precursors (C₁) and incorporated into the N-alkylamide products in a single reaction step.

Synthesis gas, commonly abbreviated as *"syngas",* is a mixture composed of carbon monoxide (CO), hydrogen (H₂) and, in some cases, carbon dioxide (CO ₂) as the main components. Synthesis gas streams may further comprise other gases such as nitrogen (N₂), helium (He), argon (Ar), water vapour (H₂O) or light hydrocarbons such as methane (CH₄), ethane (C₂H₆), propane (C₃H₈), which do not significantly alter the reactivity of the main components. Synthesis gas can be obtained from a wide range of carbonaceous sources, for example, by means of steam reforming or partial oxidation of natural gas or shale gas, coal gasification, biomass gasification and/or reforming, hydrogenation of carbon dioxide or co-electrolysis of CO₂ and water, among others, and is therefore considered a very versatile C₁ precursor for the production of chemical products from a wide range of alternative raw materials to petroleum. Fischer-Tropsch synthesis is a catalytic reaction through which syngas is directly converted into a mixture of synthetic aliphatic hydrocarbons, mainly *n-*paraffins (Fischer-Tropsch Synthesis, Catalysts, and Catalysis: Advances and Applications; B. H. Davis, M. L. Occelli (Eds), CRC Press (2016), ISBN 978-1466555297). Those skilled in the art are aware of prior work on methods in which a synthesis gas feed is mixed with a nitrogen source in gas phase, such as ammonia, and said gas mixture is put in contact with a catalyst, under standard conditions for Fischer-Tropsch synthesis, to promote the formation of organic compounds containing nitrogen, or in abbreviated form, N-compounds. Normally, a complex mixture of nitrogenous compounds is produced together with non-nitrogenous hydrocarbons.

US 2,821,537 describes a process for the catalytic hydrogenation of carbon monoxide to products containing nitrogenous organic compounds, in addition to a relatively high content of compounds containing oxygen, preferably alcohols. A synthesis gas mixture is fed, together with 0.5 to 2% by volume of a compound containing nitrogen selected from the group formed by ammonia and methylamine, and the mixture reacts on a precipitated iron catalyst. The reaction products contain nitrogenous organic compounds, more than 20% of oxygenated compounds, and a high content of non-nitrogenous olefinic hydrocarbons.

WO2009/127942A2 describes a process for the production of at least one compound containing nitrogen or phosphorus selected from linear nitriles, amides, formamides and linear compounds containing phosphorus, from synthesis gas, characterised in that the synthesis gas together with at least one compound containing nitrogen or phosphorus are introduced into a reactor and react on a catalyst, preferably composed of unsupported and promoted iron. The process is carried out at temperatures in the range of 433 K and 673 K, and pressures of between 1 and 50 bar. Ammonia, nitrogen oxides (N₂O, NO, NO₂) and mixtures thereof are described in the document as compounds containing nitrogen that can be fed, as a nitrogen source, and react together with the synthesis gas.

The organic products comprise a mixture of non-nitrogenous hydrocarbons (paraffins, olefins and oxygenates) and nitrogenous organic compounds. Alkylamides and formamides are produced with low selectivity (<1% under the conditions exemplified in the document).

A first object of the present invention is to provide a method for producing N-alkylamides by direct N-alkylation of amide compounds with synthesis gas, in a single reaction step. A second object of the present invention is to provide a method for producing fatty N-alkylamides with alkyl groups having between 7 and 30 carbon atoms.

### DESCRIPTION

The present invention relates to a method for producing N-alkylamides which can be implemented by means of at least the following steps:
i) supplying to a reactor
   a. a synthesis gas feed, and
   b. at least one amide reactant selected from among compounds with molecular formulae B₁-CONB₂H, and any of the combinations thereof, either in pure form or dissolved in a solvent,
(ii) reacting the feeds supplied in step (i) by bringing them into contact with a catalyst comprising ruthenium (Ru), either in unsupported form or dispersed on a support material, at a reaction temperature comprised between 373 and 523 degrees Kelvin (K) and at a total pressure comprised between 0.1 MPa and 30 MPa, where 1 MPa (megapascal) is equivalent to 10⁶ Pa;
iii) recovering from the reaction products of step (ii) N-alkylamides selected from those having molecular formulae B₁-CONB₂R₁, B₁-CONR₁R₂ and any combination thereof,
where the groups B₁ and B₂ can be either a hydrogen atom, identical or different hydrocarbyl groups, or any combination thereof, and the groups R₁ and R₂ are alkyl groups with *n* and *m* carbon atoms, respectively, in which *m* and *n* may be identical or different.

The term "conversion", as it is used in the context of the present invention, is expressed as a percentage and is understood as the fraction of COₓ (x=1 or 2), in the synthesis gas feed which is converted in the reactor to other compounds containing carbon.

The term "selectivity", as it is used in the context of the present invention, is defined on a carbon basis and, therefore, is understood to mean the ratio between the total molar amount of carbon in the products of interest, for example N-alkylamides, and the total molar amount of carbon in all reaction products, and is expressed on a percentage basis.

The terms "process" and "method", as used interchangeably, in the context of the present invention, are defined as the set of technical operations that involve the development of at least one chemical reaction, and which results in the deliberate modification of at least one chemical compound, which is part of a feed to the method, into at least one different chemical product.

The term "N-alkylation", as it is used in the context of the present invention, is defined as a chemical reaction in which an alkyl group is added to a nitrogen atom in a molecule. Said molecule is an amide compound according to the present invention.

### Method

The types of reactors that are suitable for implementing the method of the invention are those that allow the contact of gaseous and liquid phases with a solid catalyst. In one embodiment of the invention, the reactor operates in continuous mode, whereby at least one gas feed stream and one liquid feed stream are continuously provided to the reactor inlet, such as a slurry bubble column reactor or a trickle-bed reactor, and a product stream is continuously removed at the reactor outlet. In another embodiment of the invention, the reactor operates in batch mode, whereby the gas phase reactants and the liquid phase feeds and the catalyst are introduced into the reactor before the reactor temperature is adjusted to the reaction temperature, the reaction continues without additional streams being provided to the reactor inlet during the reaction. After a certain reaction time, the reaction ends by lowering the reactor temperature below the temperature range for the inventive method, and at least one product stream is collected from the reactor outlet. In another embodiment of the invention, the reactor operates in semi-batch mode, whereby the gas phase reactants and the liquid feeds and the catalyst are introduced into the reactor before the reactor temperature is adjusted to the reaction temperature, the reaction proceeds under the continuous provision of a gas feed stream, for example synthesis gas, to the reactor inlet. The reaction ends after a certain reaction time by lowering the reactor temperature below the temperature range for the inventive method, and at least one product stream is collected from the reactor outlet.

According to the present invention, the H₂:CO molar ratio in the synthesis gas feed can be in the range of 0.1 to 5.0. In a preferred embodiment of the present invention, said H₂:CO molar ratio in the synthesis gas is in the range of 0.3 to 3.0. More preferably, the H₂:CO molar ratio in the synthesis gas is in the range of 0.5 to 2.0.

According to the present invention, the synthesis gas feed can comprise CO₂. According to the present invention, the CO₂:CO molar ratio in the synthesis gas feed can be in the range of 0 to 2.0. In a preferred embodiment of the present invention, said CO₂:CO molar ratio in the synthesis gas is in the range of 0 to 0.5. More preferably, the CO₂:CO molar ratio in the synthesis gas is in the range of 0 to 0.1.

The synthesis gas feed can also comprise other compounds such as nitrogen, helium, argon, water, C₁ -C₄ alkanes (alkanes comprising between 1 and 4 carbon atoms) or combinations thereof. These compounds do not interfere with the conversion of the synthesis gas.

In addition to synthesis gas, in the method of the invention, at least one amide reactant is fed to the reactor. Said amide reactant can be selected from those having molecular formulae B₁-CONB₂H, and combinations thereof, where the groups B₁ and B₂ can be a hydrogen atom or identical or different hydrocarbyl groups. According to a preferred embodiment of the invention, said amide reactant is a formamide selected from those having molecular formulas H-CONH₂, H-CONB₂H and combinations thereof. According to an even more preferred embodiment of the invention, said amide reactant is N-methylformamide.

In a particular embodiment, the N-alkylamide products obtained by means of the method of the invention have a molecular formula B₁-CONB₂R₁ in which the groups B₁ and B₂ are either a hydrogen atom, identical or different hydrocarbyl groups, or any combination thereof, and the group R₁ is a linear n-alkyl group with n carbon atoms. In another particular embodiment of the method of the invention, the N-alkylamide products have a molecular formula B₁-CONHR₁ in which the group B₁ is a hydrogen atom or a hydrocarbyl group, and the group R₁ is a linear n-alkyl group with n carbon atoms. In another particular embodiment of the method of the invention, the N-alkylamide products have a molecular formula B₁-CONR₁R₂, in which the group B₁ is a hydrogen atom or a hydrocarbyl group, and the groups R₁ and R₂ are linear alkyl groups with *n* and *m* carbon atoms, respectively, in which *n* and *m* may be identical or different.

In a preferred embodiment of the invention, *n* is a number equal to or greater than 7.

In another preferred embodiment of the invention, *m* is a number equal to or greater than 7.

Said amide reactant can be supplied as a pure compound or as part of a solution, in other words, dissolved in a suitable solvent that is stable under the conditions of the method. According to a particular embodiment of the invention, said amide reactant is supplied to the reactor as part of a solution in a suitable solvent, which is stable under the conditions of the method.

The solvent supplied in step (i) provides better temperature control and a means of solubilising compounds in the reactor. Said compounds comprise the amide reactant, reaction products, metal species of the catalyst or any combination thereof. Said solvent can be selected from the list of aliphatic hydrocarbons, aromatic hydrocarbons, oxygenated organic compounds, water, nitrogenous organic compounds, halogenated organic compounds, liquid organic salts and combinations thereof. More specifically, aliphatic hydrocarbon solvents can be selected from the non-limiting list consisting of *n*-hexane, *n*-decane, *n*-dodecane, *n-*hexadecane, *iso*pentane (2-methylbutane), *iso*-hexane (2-methylpentane), *iso*-octane (2,2,4-trimethylpentane) and 2,6,11,15-tetramethylhexadecane. Again, more specifically, aromatic hydrocarbon solvents can be selected from the non-limiting list consisting of benzene, toluene, xylene, trimethylbenzenes and naphthalene. Again, more specifically, the oxygenated organic compound solvents can be selected from the non-limiting list consisting of methanol, ethanol, *iso*propanol, *n*-butanol, *n*-hexanol, 1,4-dioxane, dimethylformamide, tetrahydrofuran, di-*n*-butyl ether, cyclohexanone, methyl isobutyl ketone, ethylene glycol, propylene glycol, cyclopropyl carbonate, glycerol and ethyl acetate. Again, more specifically, the nitrogenous organic compound solvents can be selected from the non-limiting list consisting of acetonitrile, benzonitrile, diethanolamine, tri-*n*-butylamine, triethylamine, N,N-dimethylaniline, pyridine, 2-methylpyridine, N-methylmorpholine, N-methylpiperidine, N-methylpyrrole and quinoline. Again, more specifically, the halogenated organic compound solvents can be selected from the non-limiting list consisting of methylene chloride, chloroform, 1,2-dichloroethane, perfluoropentane, perfluorohexane, perfluorotoluene, monofluorobenzene and perfluorobenzene. Again, more specifically, the liquid organic salt solvents are selected from the non-limiting list consisting of salts comprising organic cations of the imidazolium, pyridinium, 1,2,3-triazolium, morpholinium, piperidinium, pyrrolidinium, pyrazolium, ammonium and phosphonium type, associated with anions of the PF⁶⁻, BF⁴⁻, NTf²⁻ (bis(trifluoromethylsulphonyl)amide), OTf⁻(trifluoromethylsulfonate), DCA⁻(dicyanamide), acetate and CH₃SO₃⁻type.

According to another embodiment of the invention, the amide compound is introduced into the reactor in the dual role of reactant and solvent. The application of a single compound in the dual function of amide reactant and solvent facilitates the design of the method, since the reaction products can be separated from the solvent and the latter recycled to the reactor, with a small replenishment addition to compensate for its consumption in the reaction. In a particular embodiment of the invention, N-methylformamide is introduced into the reactor in the dual role of reactant and solvent.

According to the present invention, the method is carried out at a reaction temperature in the range of 373 degrees Kelvin (K) to 523 K. The reaction temperature, as it is used in the context of the present invention, is understood as the maximum temperature in the reactor measured by means of a type K thermocouple located inside a stainless steel sheath and in contact with the liquid phase. In a preferred embodiment of the present invention, the method is carried out at a reaction temperature in the range of 393 K to 453 K. More preferably, the method is carried out at a reaction temperature in the range of 413 K to 433 K.

The suitable operating pressures for the method of the invention are between approximately 0.1 MPa and 30 MPa. In a preferred embodiment of the present invention, the method is carried out at a pressure of between 1 MPa and 20 MPa. In an even more preferred embodiment of the present invention, the method is carried out at a pressure of between 4 MPa and 10 MPa.

In the method of the invention, the distribution of alkyl substituents in the N-alkylamide products may obey what is known as the Anderson-Schulz-Flory (ASF) chain length distribution, which is typically observed for reactions that proceed through a chain growth polymerisation mechanism from C₁ units. This product distribution is described mathematically with the following relationship (equation 1): ${W}_{n} = n ⋅ {\left(1-α\right)}^{2} ⋅ {α}^{n - 1}$ (Equation 1) in which Wₙ is the mass fraction of the N-alkylamide products for which the alkyl group (C*ₙ*), or the longest alkyl group if more than one alkyl group is attached to the nitrogen atom of the amide group, contains *n* carbon atoms, and α (alpha) represents the probability factor of chain growth, which represents the probability, on a basis of 0 to 1, that in the polymerisation process a C₁ monomer species is added to a *Cₙ* hydrocarbon chain*,* so that the latter grows by one carbon atom. Experimentally, the probability of chain growth (alpha) can be determined according to the relationship α*=e^{m},* where e is the Euler number and m represents the slope obtained for the mathematical function of linear regression of the data in a graph representing Ln(W*ₙ*/*n*) versus *n* over a sufficiently wide range of carbon chain lengths *n,* in which Ln represents the natural logarithmic function.

Normally, the mixture of products of the inventive method is fractionated to recover N-alkylamide products. The recovery of the N-alkylamide products from mixtures with other reaction products, for example paraffins, can be carried out by means of methods such as distillation, liquid-liquid extraction, selective adsorption or reactive derivatisation of N-alkylamides to other derivative compounds, such as, for example, hydrazones, oximes, enamines, acetals or organoboron compounds, followed by any of the previous methods.

In a particular embodiment of the invention, the stream containing unconverted reactants, solvent, unrecovered reaction products, or a combination thereof, is recirculated to the reactor.

### Catalyst

According to the present invention, the catalyst is a solid. In a particular embodiment of the invention, the catalyst is a supported metal catalyst. The concept of "supported metal catalyst" is understood in the art as a catalyst composition comprising, on the one hand, a metal part, dispersed in the form of particles with an average diameter typically less than 100 nm, which is generally catalytically active, or can become an active phase *in situ,* before or during the use of the catalyst, and, on the other hand, a non-metal part, the carrier or support material, which is generally porous and forms most of the catalyst by mass.

Various methods are known in the art for incorporating a metal part into a support material. These methods include preparation techniques such as impregnation, precipitation by deposition, ion exchange, electrochemical deposition, electrostatic adsorption, fusion infiltration and co-precipitation. The documents "Handbook of Heterogeneous Catalysis", G. Ertl, H. Knózinger, F. Schüth, J. Weitkamp (Editors); volume 1, Wiley-VCH Verlag GmbH & Co. Weinheim, 2008 and "Synthesis of solid catalysts", K. P. de Jong (Editor); Wiley-VCH Verlag GmbH & Co. Weinheim, 2009 provide references to existing methods for incorporating a metal part into a support material.

According to the present invention, the solid catalyst comprises ruthenium (Ru) as the active metal. According to a preferred embodiment of the invention, Ru represents more than 50% of the metal part. More preferably, Ru represents more than 80% of the metal part.

The support material must be chemically stable under the reaction conditions found in a synthesis gas conversion method. Said support can be an oxide selected from the porous oxide-type supports used as support materials for supported metal catalysts in the art, and which can comprise among others, SiO₂, transition forms of AI₂O₃, α-Al₂O₃, TiO₂, ZrO₂, carbide or oxycarbide material such as SiC, SiOₓC_{y}, with x in the range of 0<x<2 and y in the range of 0<y<1 and any combination thereof. Alternatively, the support material can be carbon-based. Alternatively, the support material can be a compound incorporating a combination of said materials. Transition forms of AI₂O₃ are understood to be metastable polymorphs of aluminium (III) oxide that can be obtained at intermediate temperatures during the transformation of aluminium hydroxides and oxohydroxides to the most thermodynamically stable phase, that is, alpha-Al₂O₃ (α-Al₂O₃). Said transition forms of AI₂O₃ include chi-Al₂O₃, kappa-Al₂O₃, gamma-Al₂O₃, delta-Al₂O₃, theta-Al₂O₃, eta- AI₂O₃, rho-Al₂O₃, and combinations thereof.

Preferably, the loading by weight of the metal, preferably Ru, defined as the weight fraction of the total catalyst corresponding to the metal, preferably Ru, in said catalyst, is in the range of 0.5 - 50% by weight, and more preferably in the range of 3 - 20% by weight.

The metals are incorporated onto the support material using metal precursor compounds such as inorganic and organic metal salts, metal groups, and organometallic complexes. Examples of precursors containing ruthenium are ruthenium acetylacetonate, ruthenium chloride, ruthenium nitrosyl nitrate, ammonium hexachlororuthenate, triuthenium dodecacarbonyl, bis(cyclopentadienyl)ruthenium(II) and the like. The ruthenium precursors can provide ruthenium in a zero oxidation state, oxidation state II, oxidation state III, oxidation state IV, oxidation state VI, or a combination thereof. According to a preferred embodiment of the invention, the catalyst is prepared using ruthenium nitrosyl nitrate as the ruthenium precursor. Generally, after the incorporation of the metal precursor, a heat treatment is applied under a gas atmosphere, sealed or flowing, of air, water vapour, an inert gas such as nitrogen, helium, argon or combinations thereof, to decompose the metal precursor into an oxide or suboxide form on the surface of the support material.

In another particular embodiment of the invention, the catalyst is an unsupported (bulk) metal catalyst, such as a finely divided metal powder, a metal gauze or a metal foam.

In a particular embodiment of the invention, the catalyst comprises, in addition to Ru, also a second metal (M2) selected from the list of rhodium (Rh), cobalt (Co), iridium (Ir) and any combination thereof. In a preferred embodiment, the catalyst comprises, in addition to Ru, rhodium (Rh). The content of the second metal M2, expressed as the M2:Ru molar ratio, is preferably comprised between 0.001 and 1, more preferably between 0.001 and 0.5, and even more preferably between 0.001 and 0.2.

It is common in the art for supported metal catalysts to comprise what are known as promoters, in addition to the main active metal and the support material in the case of supported catalysts. Generally, a promoter is a substance that improves the performance of the catalyst in terms of activity, selectivity to a desired product or fraction of products, for example, N-alkylamides in the present invention, stability under operating conditions, or a combination thereof.

In another particular embodiment of the invention, the catalyst comprises at least one promoter. Said promoter can be selected from the list of alkali metals, alkaline earth metals, lanthanides, transition metals (other than ruthenium (Ru), rhodium (Rh), iridium (Ir) and cobalt (Co)), boron (B), aluminium (Al), gallium (Ga), indium (In), carbon (C), germanium (Ge), tin (Sn), nitrogen (N), phosphorus (P), arsenic (Ar), antimony (Sb), and any combination thereof. Any of these elements may be in elemental form or in ionic form. In a preferred embodiment of the invention, the promoter contributes to increased selectivity to N-alkylamides. In a preferred embodiment of the invention, said promoter is selected from the list of alkali metals, alkaline earth metals, lanthanides, yttrium (Y), manganese (Mn), zinc (Zn), gallium (Ga), boron (B), indium (In) and any combination thereof. In an even more preferred embodiment of the invention, said promoter is selected from the list of alkali metals, alkaline earth metals, lanthanides, Y, B, Mn and any combination thereof. Said promoter is present in a content in the range of 0 - 50% by weight, preferably in the range of 0 - 20% by weight, and more preferably in the range of 0 - 10% by weight, calculated on the basis of the total mass of the promoter element to the total mass of the catalyst.

Suitable methods for incorporating the second metal M2 and/or promoter elements include techniques known in the art such as impregnation, precipitation by deposition, ion exchange, electrochemical deposition, electrostatic adsorption, fusion infiltration or co-precipitation with suitable precursors, including inorganic and organic salts, the metal groups and organometallic complexes of the corresponding element. Examples of precursors containing rhodium are rhodium acetylacetonate, rhodium chloride, rhodium nitrate, rhodium sulphate, dirhodium carbonyl chloride, rhodium (II) acetate dimer, dicarbonyl (pentamethylcyclopentadienyl) rhodium (I), acetylacetone (1,5-cyclooctadiene) rhodium (I) and the like. The rhodium precursors can provide ruthenium in a zero oxidation state, oxidation state I, oxidation state II, oxidation state III, or a combination thereof.

Before the application thereof in the method of the invention, either with or without a support, the catalyst is normally subjected to heat treatments in the presence of hydrogen or an alternative reducing agent to convert all or part of the active metal part to the metallic state, in other words, zerovalent. In a preferred embodiment, said reduction treatment takes place at a temperature in the range of 373-873 K, and more preferably in the range of 373-773 K in the flow of a stream comprising hydrogen, either as a pure gas or in combination with an inert gas selected from the list of nitrogen, helium, argon or combinations thereof. Said heat treatment may be omitted in cases where exposure to the reaction conditions of the method of the invention, in other words, in the presence of synthesis gas as a reducing atmosphere, is sufficient to convert all or part of the active metal part to a metallic state, *in situ,* in the reactor of the method of the invention.

During step (ii) of the method of the invention, the metal of the catalyst, that is ruthenium, alone or combined with a second metal (M2) selected from the list of rhodium (Rh), cobalt (Co), iridium (Ir), and any combination thereof, can have one or more formal oxidation states in the reactor. Said metal preferably has an oxidation state of +3 or lower, more preferably an oxidation state of +2 or lower, even more preferably an oxidation state of +1 or lower, and even more preferably an oxidation state of 0, in other words, a zerovalent state.

In a particular embodiment of the invention, the catalyst is added to the reaction medium in a ratio of catalyst mass to solvent volume, alternatively volume of amide reactant when it is fed in the form of a pure compound, in the range of 0.001 kg/l to 0.1 kg/l, preferably in the range of 0.001 kg/l to 0.01 kg/l.

The present invention is described in more detail by means of the following figures and experimental examples.

### FIGURES

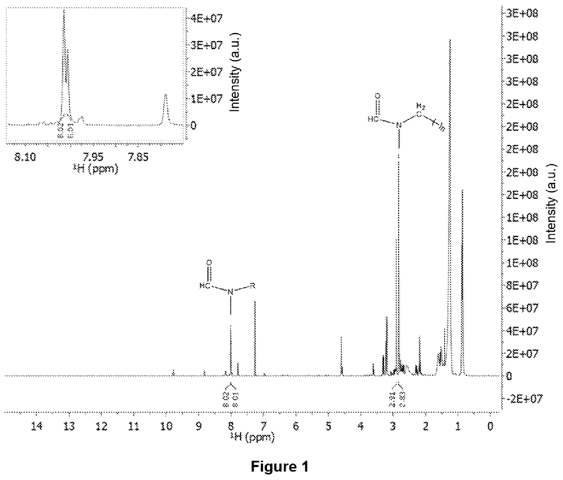

**Figure** 1: ¹³C[¹H] nuclear magnetic resonance spectrum (400 MHz, CDCl₃, room temperature) for a representative mixture of products comprising N-alkylamides obtained according to the method of the invention using N-methylformamide as the amide reactant. Method conditions: 5 ml of N-methylformamide, 82.8 mg of Ru/Al₂O₃ catalyst, T = 413K, P=1.5 MPa (initial, measured at room temperature), N₂ (99.999% vol) gaseous phase, stirring speed of 800 revolutions per minute, reaction time of 24 h. The x-axis represents the chemical shift in units of parts per million (ppm), increasing from right to left. The y-axis represents the intensity in arbitrary units, increasing from bottom to top. The inset shows an enlarged view of a spectral region. δ 8.02 - 8.01 (m, 1H), 3.32 - 3.18 (m, 2H), 2.94 - 2.81 (m, 3H), 1.64 - 1.57 (m,3H), 1.31 (d, J = 46.8 Hz, 37H), 0.90 - 0.83 (m, 10H).

### EXAMPLES

The following examples are provided by way of illustration and are not intended to limit the scope of the invention.

The following experimental methods have been used to determine the properties of the materials prepared and used in the following examples:
(i) The specific surface area and pore volume of the support materials used for the synthesis of catalysts were determined by means of nitrogen adsorption. The nitrogen adsorption isotherms were recorded using an ASAP 2420 apparatus (Micromeritics) at -196°C (77 K). Before analysis, approximately 200 mg of the sample (sieve fraction 0.2 - 0.4 mm) was degassed at a temperature of 673 K and a vacuum of ~0.5 Pa for 15 hours. The specific surface area was determined by applying the Brunauer-Emmett-Teller (BET) equation in the relative pressure range (P/P°) of 0.05 - 0.3. The pore volume was derived from nitrogen adsorption at a relative pressure P/P ⁰=0.95.
(ii) The elemental composition of the catalysts was determined by means of X-ray fluorescence spectroscopy (XRF) on a Zetium 4 kV spectrometer (Malvern Panalytical), equipped with an Rh cathode as an X-ray source. The measurements of the powder samples were taken at room temperature and under helium atmosphere. Before the measurements, the device was calibrated by preparing solid mixtures containing known metal concentrations, within the range consistent with the theoretical metal content of the catalysts. The calibration was repeated for each support material.
(iii) The molecular structure of the N-alkylamide product mixture was evaluated by means of ¹H and ¹³C nuclear magnetic resonance (NMR) spectroscopy and distortionless enhancement by polarisation transfer (DEPT-¹³C) NMR spectroscopy. Approximately 25 mg of the product mixture recovered after a reaction test was transferred to a Young tube for NMR and diluted with deuterated chloroform. The spectra were acquired on a 400 MHz NMR spectrometer (Bruker) equipped with an autosampler at a temperature of 293 K.

### Synthesis of catalysts according to the invention

### Ru/SiO₂ catalyst

A ruthenium precursor solution was prepared by dissolving ruthenium (III) nitrosyl nitrate (N₄O₁₀Ru, Alpha Aesar, Ru 31.3% minimum content) in a 0.1 M HNO₃ solution in deionised water. Specifically, 1.0 g of metal precursor was added to 2.5 ml of acid solution. Powdered silica gel (S10020M - Silicycle) was used as the catalyst support. The metal was incorporated by means of incipient wetness impregnation. The SiO₂ support was first dried in a glass flask at 423 K for 15 hours under a dynamic vacuum (1.5 mbar). Next, a volume of ruthenium precursor solution, equivalent to 90% of the pore volume of the SiO₂ support (0.72 cm³/g), with the dry support, under magnetic stirring and static vacuum. After the impregnation, the material was kept under dynamic vacuum (1.5 mbar) for at least 3 h, it was transferred to a quartz fixed-bed tubular reactor, axially mounted in a furnace, and was treated at 623 K for 3 h (heating ramp from room temperature of 1 K/min) under a flow of N₂/H₂ (80% N2, 20% H₂, vol) to decompose the Ru precursor and convert the metal to its metallic state. After the heat treatment, the tubular reactor was purged with N₂ flow and the activated catalyst was transferred to a glove box filled with N₂ (O₂<1.0 ppm, H₂O<0.1 ppm) for storage. The Ru content in the Ru/SiO₂ catalyst was 7.0% by weight.

### RU/AI₂O₃ catalyst

The RU/AI₂O₃ catalyst was synthesised as detailed above for Ru/SiO₂, using γ-AI₂O₃ as the support material in this case. The γ-AI₂O₃ support was synthesised by means of calcination of a pseudo-boehmite precursor (DISPERAL 80, Sasol) at 823 K (heating ramp of 2 K/min from room temperature) for 5 h in a muffle furnace under an air atmosphere, and presented a pore volume of 0.66 cm³/g. After the incorporation of Ru by incipient wetness impregnation, as detailed above, the solid was dried at room temperature under a dynamic vacuum (1.5 mbar) for at least 3 h, it was transferred to a quartz fixed-bed tubular reactor, axially mounted in a furnace, and was treated at 623 K for 3 h (heating ramp from room temperature of 1 K/min) under an N₂/H₂ flow (80% N₂, 20% H₂, vol) to decompose the Ru precursor and convert the metal to its metallic state. The Ru content in the Ru/AI₂O₃ catalyst was 8.0% by weight.

### General experimental method for testing catalysts according to the invention

In a general experimental method, the conversion reaction is carried out in a 316L stainless steel batch reactor (ILSHIN), magnetically stirred, with an internal volume of approximately 30 ml. The temperature reading is taken with a type K thermocouple covered with a 316L stainless steel sheath, inserted into the liquid reaction medium, while the pressure reading is performed by means of a digital pressure transducer (Sensys).

Before the reaction tests, all liquid reactants were dried for 15 hours using a thermally activated molecular sieve (5 Å, particle size 3.2 mm, Sigma Aldrich) and then stored in a glove box with N₂ atmosphere (O₂ < 1.0 ppm, H₂O <0.1 ppm). Also under exclusion of air, the powdered catalyst, the amide reactant compound, the solvent (when different from the amide reactant compound) and a magnetic stirring bar (L x D 9 mm x 20 mm) were added to a cylindrical PTFE liner, and the latter was capped and inserted into the autoclave reactor body. Next, the autoclave was sealed and purged three times with a synthetic synthesis gas mixture containing CO/H₂/Ar (Ar used as internal standard for gas chromatography analysis of gaseous products) in pre-set volumetric proportions, and then filled with the same synthesis gas mixture at a pre-set total pressure P (bar) at room temperature. The reactor was inserted into a custom-made aluminium block mounted on a stirring/heating plate (Heidolph), the stirring speed was adjusted to 800 rpm and the temperature inside the autoclave liner was raised to the reaction temperature at a rate of 3 K/min. After a selected reaction time, the autoclave reactor was cooled to room temperature. The gaseous phase in the headspace was sampled on an Agilent 7890 gas chromatograph equipped with two analysis channels and using He as the carrier gas. A first channel with a HP-PLOT-Q compact column (30 m, 0.32 mm internal diameter, 20.0 µm film thickness) and an HP-MOLSIEVE compact column molecular sieve (30 m, 0.32 mm internal diameter, 12.0 µm film thickness) and a TCD detector for the analysis of permanent gases and carbon dioxide using argon as an internal standard; and a second analysis channel equipped with a DB1 capillary column (60 m, 0.32 mm internal diameter, 3 µm film thickness) and an FID detector for the analysis of hydrocarbons and nitrogenous compounds. The liquid phase of the product was collected directly from the liner inside the reactor and filtered to remove solid particles prior to analysis. The gas chromatography analysis was performed on a Shimadzu GC-2010 Plus gas chromatograph equipped with an HP 5 capillary column (30 m, 0.25 mm internal diameter, 0.25 µm film thickness) and an FID detector, using H₂ as the carrier gas and tetrahydrofuran (THF) as the external standard. Both liquid and gaseous phase products were identified by means of gas chromatography/mass spectrometry using an Agilent 8890 gas chromatograph paired with a 5977B single quadrupole GC/MSD equipped with an HP5 capillary column (30 m, 0.32 mm internal diameter, 0.32 µm film thickness) for compound separation and an FID detector for analysis. Helium was used as the carrier gas. The mass-based response factors in the FID detector of those reaction products not available in pure form from commercial suppliers were determined using a poIyARC microreactor coupled with the FID detector in the Agilent 8890 gas chromatograph.

### Example I

In an example according to the present invention, 79.8 mg of RU/AI₂O₃ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 1:1. The reaction time was 24 h.

### Example II

In an example according to the present invention, 150 mg of RU/AI₂O₃ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 1:1. The reaction time was 24 h.

### Example III

In an example according to the present invention, 149 mg of Ru/SiO₂ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 1:1. The reaction time was 24 h.

### Example IV

In an example according to the present invention, 189 mg of Ru/SiO₂ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 1:1. The reaction time was 24 h.

### Example V

In an example according to the present invention, 149 mg of Ru/SiO₂ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 2:1. The reaction time was 24 h.

### Example VI

In an example according to the present invention, 151 mg of RU/AI₂O₃ were applied as catalyst, 1 ml of N-methylformamide (Sigma Aldrich, 99%) was added as the amide reactant dissolved in 12 ml of 1,4-dioxane (Sigma Aldrich, anhydride, 99.8%) as solvent. The method was performed according to the general experimental method for catalytic tests described above. The H₂:CO molar ratio in the feed synthesis gas was 1:1. The reaction time was 24 h.

### Example VIl

In an example according to the present invention, 200 mg of Ru/SiO₂ were used as catalyst and 12 ml of N-methylformamide (Sigma Aldrich, 99%) were added to the reactor in the dual role of amide reactant and solvent. The experiment was performed according to the general experimental method for catalytic tests described above. The H₂:CO molar ratio in the feed synthesis gas was 1:1. The reaction time was 24 h.

### Example VIII

In an example according to the present invention, 199 mg of Ru/AI₂O₃ were used as catalyst, 0.5 ml of N-methylformamide (Sigma Aldrich, 99%) was added as the amide reactant dissolved in 12 ml of 1,4-dioxane (Sigma Aldrich, anhydride, 99.8%) as solvent. The experiment was performed according to the general experimental method for catalytic tests described above. The H₂:CO molar ratio in the feed synthesis gas was 1:1. The reaction time was 24 h.

**Table 1: Summary of conversion and product selectivities for the examples carried out according to the present invention.**

| **Example** | **T^{(a)} (K)** | **P^{(b)} (MPa)** | **CO:M^{(c)} (-)** | **X_{CO}^{(d)} (%)** | **Selectivity (C%)^{(e)}** | | | | **α^{(h)} (-)** |
|---|---|---|---|---|---|---|---|---|---|
| | | | | | **CO₂** | **Hydrocarbons^{(f)}** | **N-alkylamides** | **Others^{(g)}** | |
| I | 413 | 3.6 | 140:1 | 19.2 | 19.2 | 8.1 | 14.2 | 58.5 | 0.89 |
| II | 433 | 4.3 | 80:1 | 72.7 | 30.5 | 2.5 | 11.6 | 55.4 | 0.87 |
| III | 413 | 3.9 | 90:1 | 38.9 | 5.9 | 17.5 | 33.0 | 43.6 | 0.91 |
| IV | 433 | 3.9 | 70:1 | 77.8 | 20.5 | 10.2 | 18.8 | 50.5 | 0.82 |
| V | 413 | 3.8 | 60:1 | 46.5 | 4.3 | 33.1 | 28.4 | 34.2 | 0.82 |
| VI | 413 | 4.0 | 70:1 | 18.2 | 10.9 | 49.7 | 38.8 | 0.6 | 0.88 |
| VII | 413 | 6.6 | 100:1 | 23.0 | 9.9 | 20.6 | 42.4 | 27.1 | 0.85 |
| VIII | 413 | 6.9 | 100:1 | 6.9 | 19.6 | 40.0 | 39.6 | 0.0 | 0.79 |

(a) Reaction temperature; (b) Total pressure, determined at the beginning of the reaction and at the reaction temperature; (c) Molar ratio of CO in the synthesis gas feed with respect to the total metal in the catalyst; (d) Conversion of CO; (e) Product selectivity, determined on a molar basis of carbon; (f) Non-nitrogenous hydrocarbons, in other words, paraffins and olefins; (g) By-products of the conversion of the amide reactant; (I) Chain growth probability parameter determined from analysis of alkyl chain length in N-alkylamide products according to an ASF-type distribution in the chain length range n=5-20.

Although the present invention has been described in terms of preferred embodiments, it is understood that said description is not to be construed as a limitation of the invention described herein. Upon reading the description, it will be immediately apparent to those with ordinary skill in the field, in view of the teachings of this invention, that various alterations and modifications can be made to it. The appended claims are to be construed as encompassing all alterations and modifications falling within the spirit and scope of the present invention.

## Claims

1. A method for producing N-alkylamides comprising at least the following steps:
i) supplying to a reactor
c. a synthesis gas feed, and
d. at least one amide reactant selected from among compounds with molecular formulae B₁-CONB₂H, and any of the combinations thereof.
(ii) reacting the feeds supplied in step (i) by bringing them into contact with a catalyst comprising ruthenium (Ru), either in unsupported form or dispersed on a support material, at a reaction temperature comprised between 373 and 523 degrees Kelvin and at a total pressure comprised between 0.1 MPa and 30 MPa;
iii) recovering from the reaction products of step (ii) N-alkylamides selected from those having molecular formulae B₁-CONB₂R₁, B₁-CONR₁R₂ and any combination thereof,
where the groups B₁ and B₂ are either a hydrogen atom, identical or different hydrocarbyl groups, or any combination thereof, and the groups R₁ and R₂ are alkyl groups with *n* and *m* carbon atoms, respectively, in which *m* and *n* may be identical or different.

2. The method according to claim 1, in which the catalyst is a supported catalyst in which Ru is dispersed on a support material selected from the list comprising SiO₂, transition forms of Al₂O₃, α-Al₂O₃, TiO₂, ZrO₂, carbide or oxycarbide materials selected from: SiC, SiOₓC_{y}, x being in the range of 0<x<2 and y being in the range of 0<y<1, carbon and any combination thereof.

3. The method according to any of the preceding claims, in which the weight fraction of the total catalyst corresponding to Ru is in the range of 0.5 - 50% by weight.

4. The method according to any of the preceding claims, in which the catalyst also comprises a second metal M2 selected from the list of rhodium (Rh), cobalt (Co), iridium (Ir) and any combination thereof.

5. The method according to claim 4, in which the content of the second metal (M2), expressed as the M2:Ru molar ratio, is comprised between 0.001 and 1.

6. The method according to any of the preceding claims, in which the catalyst also comprises a promoter element selected from the list comprising: alkali metals, alkaline earth metals, lanthanides, transition metals (other than ruthenium (Ru), rhodium (Rh), iridium (Ir), cobalt (Co)) boron (B), aluminium (Al), gallium (Ga), indium (In), carbon (C), germanium (Ge), tin (Sn), nitrogen (N), phosphorus (P), arsenic (Ar), antimony (Sb), and any combination thereof.

7. The method according to claim 6, in which the promoter element is present in a content of between 0 and 50% by weight, calculated on the basis of the total mass of the promoter element with respect to the total mass of the catalyst.

8. The method according to any of the preceding claims, in which in step i, d) a solvent is added selected from the list comprising: aliphatic hydrocarbons, aromatic hydrocarbons, oxygenated organic compounds, water, nitrogenous organic compounds, halogenated organic compounds, liquid organic salts and combinations thereof.

9. The method according to any of the preceding claims, in which the reaction is carried out in a stirred batch reactor.

10. The method according to any of claims 1 to 8, in which the reaction is carried out in a continuous reactor to which both a gas stream and a liquid stream are fed.

11. The method according to any of the preceding claims, in which the reaction temperature is in the range of between 393 K and 453 K.

12. The method according to any of the preceding claims, in which the reaction pressure is in the range of 1 MPa to 20 MPa.

13. The method according to any of the preceding claims, in which the H₂:CO molar ratio in the synthesis gas feed is between 0.1 and 5.0.

14. The method according to any of the preceding claims, in which the N-alkylamide products have a molecular formula B₁-CONB₂R₁ in which the groups B₁ and B₂ are either a hydrogen atom, identical or different hydrocarbyl groups, or any combination thereof, and the group R₁ is a linear *n*-alkyl group with *n* carbon atoms.

15. The method according to any of claim 14, in which the group B₂ is a hydrogen atom.

16. The method according to any of claims 1 to 13, in which the N-alkylamide products have the molecular formula B₁-CONR₁R₂, in which the group B₁ is a hydrogen atom or a hydrocarbyl group, and the groups R₁ and R₂ are linear *n*-alkyl groups with *n* and *m* carbon atoms, respectively, in which *m* and *n* may be identical or different.

17. The method according to any of claims 14 to 16, in which *n* is a number equal to or greater than 7.

18. The method according to claim 16, in which m is a number equal to or greater than 7.

19. The method according to any of the preceding claims, in which the synthesis gas feed additionally comprises other compounds such as nitrogen, helium, argon, water, C₁-C₄ alkanes or combinations thereof.

20. The method according to any of the preceding claims, in which after the recovery of N-alkylamides from the reaction products, a stream containing unconverted reactants, solvent, unrecovered reaction products, or a combination thereof, is recirculated to the reactor.
